(19) Europäisches Patentamt
European Patent Office
Office européen des brevets

(11) Numéro de publication : **0 465 339 B1**

(12) # FASCICULE DE BREVET EUROPEEN

(45) Date de publication du fascicule du brevet : **29.12.93 Bulletin 93/52**

(51) Int. Cl.$^5$ : **A61K 7/13**

(21) Numéro de dépôt : **91401801.5**

(22) Date de dépôt : **02.07.91**

(54) **Procédé de teinture des fibres kératiniques avec des dérivés du 4-hydroxyindole à pH acide et compositions.**

(30) Priorité : **05.07.90 FR 9008570**

(43) Date de publication de la demande : **08.01.92 Bulletin 92/02**

(45) Mention de la délivrance du brevet : **29.12.93 Bulletin 93/52**

(84) Etats contractants désignés : **AT BE CH DE DK ES FR GB GR IT LI NL SE**

(56) Documents cités :
**EP-A- 0 428 441**
**FR-A- 2 636 237**
**GB-A- 2 211 517**

(73) Titulaire : **L'OREAL**
**14, Rue Royale**
**F-75008 Paris (FR)**

(72) Inventeur : **Cotteret, Jean**
**15, allée des Meunieurs**
**F-78480 Verneuil-sur-Seine (FR)**
Inventeur : **Audousset, Marie Pascale**
**106, rue Baudin**
**F-92300 Levallois-Perret (FR)**

(74) Mandataire : **Casalonga, Axel et al**
**BUREAU D.A. CASALONGA - JOSSE**
**Morassistrasse 8**
**D-80469 München (DE)**

EP 0 465 339 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Procédé de teinture des fibres kératiniques avec des dérivés du 4-hydroxyindole à pH acide et compositions mises en oeuvre.

La présente invention est relative à un procédé de teinture des fibres kératiniques, en particulier des fibres kératiniques humaines, mettant en oeuvre des dérivés du 4-hydroxindole en association avec des bases d'oxydation et un agent oxydant en milieu acide et aux compositions mises en oeuvre au cours de ce procédé.

Il est connu de teindre les fibres kératiniques et en particulier les cheveux humains avec des compositions tinctoriales contenant en milieu alcalin des précurseurs de colorants d'oxydation et en particulier des p-phénylénediamines, des ortho ou para-aminophénols appelés généralement "bases d'oxydation".

On sait également que l'on peut faire varier les nuances obtenues avec ces bases d'oxydation en les associant à des coupleurs encore appelés modificateurs de coloration, choisis notamment parmi les métadiamines aromatiques, les méta-aminophénols et les méta-diphénols.

La demanderesse vient de découvrir que l'utilisation de dérivés du 4-hydroxyindole associés à des bases d'oxydation lorsque cette association était appliquée en présence d'un agent et à pH acide sur les cheveux, conduisait à des colorations présentant une puissance tinctoriale améliorée. Les colorations ainsi obtenues présentent par ailleurs une excellente ténacité à la lumière, aux lavages, à la transpiration et aux intempéries.

Ces résultats sont particulièrement surprenants lorsqu'on les compare à ceux obtenus avec des coupleurs classiques de la série benzénique mentionnés ci-dessus où l'on constate souvent une perte de puissance tinctoriale et une ténacité plus faible, lorsqu'on opère à pH acide.

La présente invention a donc pour objet un procédé de teinture des fibres kératiniques, en particulier des fibres kératiniques humains telles que les cheveux, comprenant l'application sur ces fibres d'au moins une composition contenant un composé hydroxyindolique de formule (I) telle que définie ci-après, un précurseur de colorant d'oxydation encore appelé base d'oxydation et un agent oxydant, à pH acide.

L'invention a également pour object un agent de teinture à deux composants, dont l'un des composants comprend le dérivé de 4-hydroxyindole et le précurseur du colorant d'oxydation et l'autre l'agent oxydant à un pH acide, et en des quantités telles que le mélange présente un pH acide.

L'invention a également pour objet la composition prête à l'emploi, contenant les différents agents utilisés pour la teinture, en milieu acide, des cheveux.

D'autres objets de l'invention apparaitront à la lecture de la description et des exemples qui suivent.

Le procédé de teinture des fibres kératiniques et en particulier des fibres kératiniques humaines telles que les cheveux, conforme à l'invention, est essentiellement caractérisé par le fait que l'on applique sur ces fibres une composition contenant dans un milieu approprié pour la teinture, au moins un coupleur répondant à la formule :

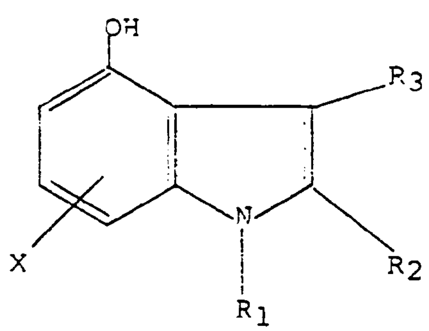

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle en $C_1$-$C_4$, un radical carboxyle ou un radical alcoxycarbonyle; X désigne un atome d'hydrogène, un radical alkyle en $C_1$-$C_4$, un radical alcoxy en $C_1$-$C_{18}$, un atome d'halogène, un groupement acétylamino; au moins l'un des groupements X, $R_1$, $R_2$, $R_3$ étant différent de l'hydrogène;

ainsi que les sels de ces composés;

au moins un précurseur de colorant d'oxydation ou base d'oxydation;

au moins un agent oxydant; le pH de la composition appliquée sur les fibres étant inférieur à 7.

Les composés préférés répondant à la formule (I), utilisés conformément à l'invention, sont les composés dans lesquels le radical alkyle désigne méthyle, éthyle; le radical alcoxycarbonyle désigne méthoxy ou éthoxycarbonyle.

Parmi ces composés, on peut citer le 4-hydroxy 5-éthoxyindole, le 4-hydroxy 5-méthoxy indole, le 4-hydroxy 1-méthyl 5-éthoxyindole, le 4-hydroxy 2-éthoxycarbonyl 5-éthoxyindole, le 4-hydroxy 2-méthyl 5-éthoxyindole, le 4-hydroxy 7-méthoxy 2,3-diméthylindole, le 4-hydroxy 5-méthyl indole.

Les sels sont choisis plus particulièrement parmi les chlorhydrates ou bromhydrates.

Les précurseurs de colorants d'oxydation ou bases d'oxydation sont des composés connus qui ne sont pas des colorants en eux-mêmes et qui forment un colorant par un processus de condensation oxydative, soit sur eux-mêmes, soit en présence d'un coupleur ou modificateur. Ces composés comportent généralement un noyau aromatique portant des groupements fonctionnels, constitués : soit par deux groupements amino; soit par un groupement amino et un groupement hydroxy; ces groupements étant en position para ou ortho, l'un par rapport à l'autre.

Les précurseurs de colorants d'oxydation de type para, utilisés conformément à l'invention, sont choisis plus particulièrement parmi les paraphénylène diamines, les para-aminophénols, les précurseurs hétérocycliques para, comme la 2,5-diaminopyridine, la 2-hydroxy 5-aminopyridine, la 2,4,5,6-tétraamino pyrimidine.

Parmi les paraphénylènediamiques, on peut citer les composés répondant à la formule (II) :

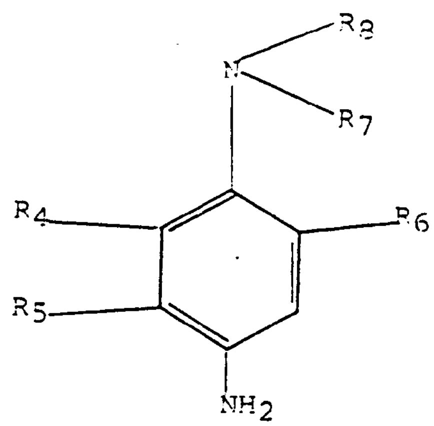

(II)

dans laquelle $R_4$, $R_5$ et $R_6$, identiques ou différents, représentent un atome d'hydrogène ou d'halogène, un radical alkyle ayant de 1 à 4 atomes de carbone, un radical alcoxy ayant de 1 à 4 atomes de carbone; $R_7$ et $R_8$, identiques ou différents, représentent un atome d'hydrogène, un radical alkyle, hydroxyalkyle, alcoxy alkyle, carbamylalkyle, mésylaminoalkyle, acétylamino alkyle, uréidoalkyle, carbalcoxyaminoalkyle, pipéridino alkyle, morpholinoalkyle; ces groupes alkyle ou alcoxy ayant de 1 à 4 atomes de carbone, ou bien $R_7$ et $R_6$ forment, conjointement avec l'atome d'azote auquel ils sont liés, un hétérocycle pipéridino ou morpholino, sous réserve que $R_4$ ou $R_6$ représente un atome d'hydrogène lorsque $R_7$ et $R_6$ ne représentent pas un atome d'hydrogène, ainsi que les sels de ces composés.

Parmi les composés particulièrement préférés répondant à la formule (II), on peut citer la p-phénylènediamine, la 2-méthyl-p-phénylènediamine, la méthoxyparaphénylènediamine, la chloroparaphénylène diamine, la 2,6-diméthylparaphénylènediamine, la 2,5-diméthylparaphénylènediamine, la 2,3-diméthyl paraphénylènediamine, la 2-méthyl 5-méthoxypara phénylènediamine, la 2,6-diméthyl 5-méthoxypara phénylènediamine, la N,N-diméthylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl] morpholine, la N-[(4'-amino)phényl]pipéridine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)

4-aminophénol, le 2-méthoxy 4-aminophénol, le 3-méthoxy 4-aminophénol, le 2,5-diméthyl 4-aminophénol, le 2-méthoxyméthyl 4-aminophénol.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène et les orthophénylènediamines.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

Les compositions de l'invention ne contiennent pas d'ions iodures ni d'ions nitrites en quantité suffisante pour oxyder le dérivé indolique de formule (I) et le précurseur de colorant d'oxydation.

Le pH de la composition appliqué sur les fibres kératiniques, en particulier les cheveux, a une valeur inférieure à 7 et est compris de préférence entre 3 et 6,9. Ce pH est ajusté par l'utilisation d'agents acidifiants bien connus dans le domaine de la teinture des fibres kératiniques, et en particulier des cheveux humains, tels que des acides minéraux comme l'acide chlorhydrique ou l'acide phosphorique, ou organiques comme les acides carboxyliques tels que l'acide tartrique ou citrique.

Les composés de formule (I) sont présents dans la composition appliquée sur les fibres kératiniques, dans des proportions comprises de préférence entre 0,01 et 3,5% en poids par rapport au poids total de la composition.

Les compositions, définies ci-dessus, appliquées dans la teinture des fibres kératiniques, peuvent également contenir en plus des coupleurs hétérocycliques de formule (I), d'autres coupleurs connus en eux-mêmes tels que des métadiphénols, des métaaminophénols, des métaphénylènediamines, des méta N-acylaminophénols, des métauréidophénols, des métacarbalcoxyaminophénols, l'$\alpha$-naphtol, des coupleurs possédant un groupement méthylène actif, tels que les composés dicétoniques, les pyrazolones.

Parmi ces coupleurs pouvant être utilisés en plus des coupleurs de formule (I), on peut citer le 2,4-dihydroxyphénoxyéthanol, le 2,4-dihydroxyanisole, le métaaminophénol, la résorcine, le monométhyléther de résorcine, la 2-méthylrésorcine, le pyrocatechol, le 2-méthyl 5-N-($\beta$-hydroxyéthyl)aminophénol, le 2-méthyl 5-N-($\beta$-mésylaminoéthyl)aminophénol, la 6-hydroxybenzo morpholine, le 2,4-diaminoanisole, le 2,4-diamino phénoxyéthanol, la 6-aminobenzomorpholine, le [2-N-($\beta$-hydroxyéthyl)amino 4-amino]-phénoxyéthanol, le 2-amino 4-N-($\beta$-hydroxyéthyl)aminoanisole, le (2,4-diamino)phényl-$\beta$, $\gamma$-dihydroxypropyléther, la 2,4-diaminophénoxyéthylamine, le 1,3-diméthoxy 2,4-diméthoxy 2,4-diamino benzène, le 2-méthyl 5-aminophénol, le 2,6-diméthyl 3-aminophénol, le 3,4-méthylènedioxyphénol, la 3,4-méthylènedioxyaniline et leurs sels.

Les compositions tinctoriales de l'invention peuvent contenir également des colorants directs tels que les dérivés nitrés de la série benzénique, les azoïques, les anthraquinoniques, le triphénylméthane et ses dérivés, les colorants xanthéniques ou aziniques. Elles peuvent contenir également des colorants d'oxydation rapides.

Ces compositions peuvent également contenir des agents tensio-actifs anioniques, cationiques, non ioniques, amphotères ou leurs mélanges.

Parmi ces agents tensio-actifs, on peut citer les alkylbenzènesulfonates, les alkylnaphtalène sulfonates, les sulfates, les éthersulfates et les sulfonates d'alcools gras, les sels d'ammonium quaternaires tels que le bromure de triméthylcétyl ammonium, le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les compositions tinctoriales sont généralement aqueuses, mais elles peuvent également contenir des solvants organiques pour solubiliser des composés qui ne seraient pas suffisamment solubles dans l'eau. Parmi ces solvants, on peut citer à titre d'exemple, les alcanols inférieurs en $C_2$-$C_4$ tels que l'éthanol et l'isopropanol, le glycérol, les glycols ou éthers de glycols, comme le butoxy-2 éthanol, l'éthylène glycol, le propylèneglycol, le monoéthyléther et le monométhyléther du diéthylèneglycol, le monoéthyléther et le monométhyléther du propylèneglycol, ainsi que les alcools aromatiques comme l'alcool benzylique ou le phénoxyéthanol, ou les mélanges de ces solvants.

La composition appliquée sur les cheveux peut également renfermer des agents épaississants choisis en particulier parmi l'alginate de sodium, la gomme arabique, les dérivés de cellulose tels que la méthylcellulose, l'hydroxyéthylcellulose, l'hydroxypropylcellulose, l'hydroxyméthylcellulose, la carboxyméthylcellulose, les polymères d'acide acrylique éventuellement réticulés, la gomme de xanthane. On peut également utiliser des agents épaississants minéraux tels que la bentonite.

La composition peut également renfermer les agents antioxydants choisis en particulier parmi le sulfite de sodium, l'acide thioglycolique, le bisulfite de sodium, l'acide ascorbique et l'hydroquinone, ainsi que d'autres adjuvants cosmétiquement acceptables lorsque la composition est destinée à être utilisée pour la teinture des fibres kératiniques humaines, tels que des agents de pénétration, des agents séquestrants, des conservateurs, des tampons, des parfums, etc...

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

L'invention a également pour objet la composition prête à l'emploi utilisée dans le procédé défini ci-dessus.

Selon une forme de réalisation préférée, le procédé comporte une étape préliminaire consistant à stocker sous forme séparée, d'une part, la composition contenant dans un milieu approprié pour la teinture le coupleur répondant à la formule (I) définie ci-dessus et les précurseurs de colorants par oxydation sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus.

La composition appliquée sur les fibres kératiniques résulte d'un mélange de 10 à 90% du composant (A) avec 90 à 10% du composant (B) contenant un agent oxydant.

L'invention a également pour objet un agent de teinture des fibres kératiniques, en particulier des cheveux, essentiellement caractérisé par le fait qu'il comporte au moins deux composants, l'un des composants étant constitué par le composant (A) défini ci-dessus et l'autre étant constitué par le composant (B) également défini ci-dessus, le pH des composants (A) et (B) étant tel qu'après mélange dans des proportions de 90 à 10% pour le composant (A) et de 10 à 90% pour le composant (B), la composition résultante ait un pH inférieur à 7.

Le composant (A) ne contient pas d'ions iodures ni d'ions nitrites en quantité suffisante pour oxyder le dérivé indolique de formule (I) et le précurseur de colorant d'oxydation.

Dans cette forme de réalisation, le composant (A) qui renferme au moins le coupleur de formule (I) et un précurseur de colorant d'oxydation, peut avoir un pH compris entre 3 et 10,5 et peut être ajusté à la valeur choisie au moyen d'agents alcalinisants habituellement utilisés en teinture des fibres kératiniques, tels que l'ammoniaque, les carbonates alcalins, les alcanolamines tels que les mono-, di- et triéthanolamines ainsi que leurs dérivés ou des agents acidifiants classiques, tels que les acides minéraux comme l'acide chlorhydrique ou l'acide phosphorique, ou organiques comme les acides carboxyliques tels que l'acide tartrique ou citrique.

Cette composition peut renfermer les différents autres adjuvants mentionnés ci-dessus, notamment des coupleurs différents des coupleurs dérivés du 4-hydroxyindole répondant à la formule (I) déjà mentionnée ci-dessus.

L'ensemble des précurseurs de colorants par oxydation de type para et/ou ortho ainsi que les coupleurs, sont présents dans des proportions comprises de préférence entre 0,05 et 7% en poids par rapport au poids total du composant (A). La concentration en composés de formule (I) peut varier entre 0,01 et 4% en poids par rapport au poids total du composant A.

Les agents tensio-actifs sont présents dans le composant (A) dans des proportions de 0,1 à 55% en poids. Lorsque le milieu contient des solvants en plus de l'eau, ceux-ci sont présents dans des proportions comprises entre 0,5 et 40% en poids et en particulier entre 5 et 30% en poids par rapport au poids total du composant (A). Les agents épaississants sont présents de préférence dans des proportions comprises entre 0,1 et 5%, et en particulier entre 0,2 et 3% en poids. Les agents anti-oxydants mentionnés ci-dessus sont de préférence présents dans le composant (A) dans des proportions comprises entre 0,02 et 1,5% en poids par rapport au poids total du composant (A).

Le composant (B) renfermant l'agent oxydant tel que défini ci-dessus, a un pH inférieur à 7. Ce pH peut avoir une valeur minimum de 1 et il est de préférence compris entre 1,5 et 3,5. Ce composant (B) peut être acidifié avec le même type d'agents acidifiants que ceux utilisés pour le composant (A).

Il peut se présenter sous forme de liquide plus ou moins épaissi, de lait ou de gel.

Cet agent de teinture à deux composants peut être conditionné dans un dispositif à plusieurs compartiments ou kit de teinture, ou tout autre système de conditionnement à plusieurs compartiments dont l'un renferme le composant (A) et le second compartiment renferme le composant (B); ces dispositifs pouvant être équipés d'un moyen permettant de délivrer sur les cheveux le mélange souhaité, tel que les dispositifs décrits dans le brevet US-A-4 823 985 de la demanderesse.

L'invention a également pour objet l'utilisation comme coupleur de dérivés du 4-hydroxy indole répondant à la formule (I) pour la teinture en milieu acide des fibres kératiniques, en association avec des précurseurs de colorants d'oxydation.

Conformément à l'invention, le procédé de teinture consiste à appliquer sur les cheveux le mélange obtenu, à le laisser poser pendant 3 à 40 minutes, puis à rincer les cheveux et éventuellement effectuer un shampooing.

Il est également possible, conformément à l'invention, d'appliquer séparément une composition contenant le coupleur de formule (I), le précurseur de colorant d'oxydation et l'agent oxydant, de façon à ce que le mélange se formant in-situ au niveau des fibres ait un pH inférieur à 7, comme défini ci-dessus.

Les exemples suivants sont destinés à illustrer l'invention sans pour autant présenter un caractère limitatif.

EXEMPLES 1 à 6

On procède à la teinture des cheveux en appliquant sur des cheveux gris à 90% de blancs un mélange extemporané de la composition colorante (A) et de la composition oxydante (B).

Ce mélange présente le pH indiqué dans le tableau des exemples qui suivent. On laisse agir ce mélange pendant 30 minutes, puis on rince les cheveux, on effectue un shampooing. Après séchage, les cheveux sont teints dans la nuance précisée au bas du tableau.

| en g | 1 | 2 | 3 |
|---|---|---|---|
| A) Composition colorante<br>2,3-diméthyl 7-méthoxy 4-hydroxyindole | 0,573 | 0,191 | 0,573 |
| 2,6-diméthylparaphénylènediamine, 2HCl | | | 0,657 |
| Paraphénylènediamine | | 0,324 | |
| Paraaminophénol | 0,327 | | |
| 2-méthyl 5-N-(β-hydroxyéthyl)aminophénol | | 0,167 | |
| Monoéthanolamine qs pH | 9,2 | 9,9 | 8,9 |
| Support 1 | | X | |
| Support 2 | X | | X |
| Eau qsp | 100 | 100 | 100 |
| B) Composition oxydante<br>Solution d'eau oxygénée à 20 volumes | | | |
| Acide phosphorique qs pH | 1,2 | 1,3 | 1,2 |
| pH mélange p/p A + B | 6,5 | | 6,4 |
| pH mélange 1/3 A + 2/3 B | | 4,4 | |
| Nuances obtenues : | blond clair irisé | blond clair cendré | gris |

| en g | 4 | 5 | 6 |
|---|---|---|---|
| A) <u>Composition colorante</u><br>2,3-diméthyl 7-méthoxy 4-hydroxyindole | 0,191 | | |
| 4-hydroxy 5-méthoxyindole | | 0,489 | 0,489 |
| 2,6-diméthylparaphénylènediamine, 2HCl | | 0,657 | |
| Paraphénylènediamine | | | 0,324 |
| 2-méthyl paraphénylènediamine, 2HCl | 0,366 | | |
| Métaaminophénol | 0,109 | | |
| α-naphtol | 0,144 | | |
| Monoéthanolamine qs pH | 9,7 | 8,9 | 9,1 |
| Support 1 | X | | |
| Support 2 | | X | X |
| Eau qsp | 100 | 100 | 100 |
| B) <u>Composition oxydante</u><br>Solution d'eau oxygénée à 20 volumes | | | |
| Acide phosphorique qs pH | 1,3 | 1,2 | 1,2 |
| pH mélange p/p A + B | 6,0 | 6,4 | 6,5 |
| <u>Nuances obtenues</u> : | blond très clair mat | pourpre | pourpre rouge |

EXEMPLE 7

COMPOSITION COLORANTE

- 2,5-diaminonitrobenzène 0,3 g
- 4-hydroxy 5-éthoxyindole 0,3 g
- Paraphénylènediamine 0,4 g

- 1-méthyl 2-hydroxy 4-aminobenzène 0,1 g
- Lauryléthersulfate de sodium à 2 moles d'oxyde d'éthylène, vendu à 28% de MA 4,2 g MA
- Nonylphénol oxyéthyléné à 9 moles d'oxide d'éthylène 1,0 g
- Monobutyléther d'éthylèneglycol 9,5 g
- Monoéthanolamine qs pH=8,4
- Métabisulfite de sodium à 35% de MA 0,45 g MA
- Séquestrant qs
- Eau qsp 100 g

Mélange poids pour poids avec une composition oxydante : solution d'eau oxygénée à 20 volumes dont le pH est ajusté à 1,3 par l'acide phosphorique.
pH spontané du mélange : 6,3.
Ce mélange est appliqué pendant 30 minutes sur des cheveux gris permanentés. Après rinçage et shampooing, les cheveux sont colorés en châtain cendré rouge.

EXEMPLE 8

COMPOSITION COLORANTE

- 4-hydroxy 5-méthylindole 0,45 g
- 2,6-diméthyl 1,4-diaminobenzène 2HCl 0,64 g
- Support 2
- Monoéthanolamine qs pH=8,9
- Eau qsp 100 g

COMPOSITION OXYDANTE

Solution d'eau oxygénée à 20 volumes ajustée à pH 1,2 par de l'acide phosphorique.
On procède à la teinture des cheveux gris à 90% de blancs en appliquant un mélange poids pour poids de la composition colorante et de la composition oxydante. Le pH du mélange est égal à 6,4. Après 30 minutes de pose, les cheveux sont rincés, lavés au shampooing, rincés une nouvelle fois puis séchés. Ils sont colorés en violine irisé.

SUPPORT DE COLORATION 1

- Nonylphénol à 4 moles d'oxyde d'éthylène, vendu sous la dénomination SINNOPAL NP4 par la Société HENKEL 25,5 g
- Nonylphénol à 9 moles d'oxyde d'éthylène, vendu sous la dénomination SINNOPAL NP9 par la Société HENKEL 17,5 g
- Monoéthyléther d'éthylèneglycol 7,0 g
- Propylèneglycol 10,5 g
- Dipropylèneglycol 0,5 g
- Alcool éthylique 2,0 g
- Lauryl éther sulfate de monoéthanolamine, vendu sous la dénomination SACTIPON 2 OM 29 par la Société LEVER à 28% de MA 4,2 g MA
- Alkyl éther sulfate de sodium à 28% de MA 0,8 g MA
- Métabisulfite de sodium en solution aqueuse à 35% de MA 0,45 g MA
- Acétate de sodium 0,8 g
- Antioxydant, séquestrant qs

SUPPORT DE COLORATION 2

- Alcool oléique polyglycérolé à 2 moles de glycérol 4,0 g
- Alcool oléique polyglycérolé à 4 moles de glycérol à 78% de MA 5,69 g MA
- Acide oléique 3,0 g
- Amine oléique à 2 moles d'oxyde d'éthylène, vendue sous la dénomination ETHOMEEN O 12 par la Société AKZO 7,0 g
- Laurylamino succinamate de diéthylaminopropyle, sel de sodium à 55% de MA 3,0 g MA
- Alcool oléique 5,0 g

- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse à 35% de MA 0,45 g MA
- Acétate d'ammonium 0,8 g
- Antioxydant, séquestrant qs

## Revendications

1. Procédé de teinture des fibres kératiniques, en particulier des fibres kératiniques humaines telles que les cheveux, caractérisé par le fait qu'on applique sur ces fibres une composition contenant dans un milieu approprié pour la teinture, au moins un coupleur répondant à la formule :

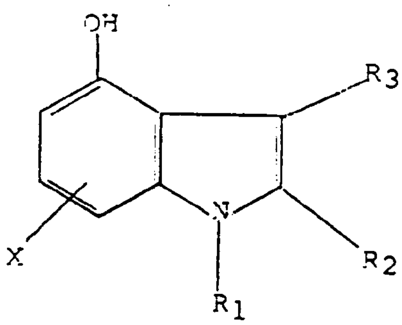

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle en $C_1$-$C_4$, un radical carboxyle ou un radical alcoxycarbonyle; X désigne un atome d'hydrogène, un radical alkyle en $C_1$-$C_4$, un radical alcoxy en $C_1$-$C_{18}$, un atome d'halogène, un groupement acétylamino; au moins l'un des groupements X, $R_1$, $R_2$, $R_3$ étant différent de l'hydrogène;
ainsi que les sels de ces composés;
   - au moins un précurseur de colorant d'oxydation;
   - au moins un agent oxydant;
   le pH de la composition appliquée sur les fibres étant inférieur à 7.

2. Procédé selon la revendication 1, caractérisé par le fait que les composés de formule (I) sont choisis parmi les composés dans lesquels le radical alkyle désigne méthyle, éthyle; le radical alcoxy carbonyle désigne méthoxy ou éthoxycarbonyle.

3. Procédé selon la revendication 2, caractérisé par le fait que les composés de formule (I) sont choisis parmi le 4-hydroxy 5-méthoxyindole, le 4-hydroxy 2-éthoxycarbonyl 5-éthoxyindole, le 4-hydroxy 2-méthyl 5-éthoxyindole, le 4-hydroxy 7-méthoxy 2,3-diméthylindole, le 4-hydroxy 5-méthyl indole, le 4-hydroxy 5-éthoxyindole, le 4-hydroxy 1-méthyl 5-éthoxyindole.

4. Procédé selon l'une quelconque des revendications 1 à 3, caractérisé par le fait que les précurseurs de colorants d'oxydation sont choisis parmi les paraphénylènediamines, les para-aminophénols, les précurseurs hétérocycliques para.

5. Procédé selon la revendication 4, caractérisé par le fait que les paraphénylènediamines sont choisies parmi les composés répondant à la formule :

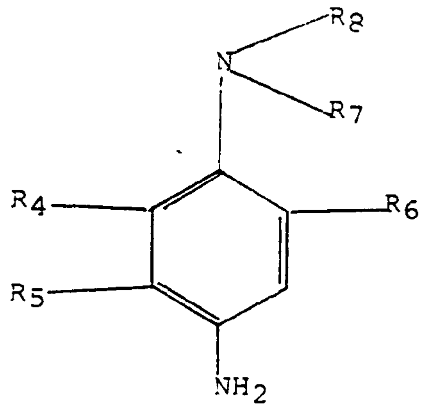

(II)

dans laquelle $R_4$, $R_5$ et $R_5$, identiques ou différents, représentent un atome d'hydrogène ou d'halogène, un radical alkyle ayant de 1 à 4 atomes de carbone, un radical alcoxy ayant de 1 à 4 atomes de carbone; $R_7$ et $R_5$, identiques ou différents, représentent un atome d'hydrogène, un radical alkyle, hydroxyalkyle, alcoxy alkyl, carbamylalkyle, mésylaminoalkyle, acétylamino alkyle, uréidoalkyle, carbalcoxyaminoalkyle, pipéridino alkyle, morpholinoalkyle; ces groupes alkyle ou alcoxy ayant de 1 à 4 atomes de carbone, ou bien $R_7$ et $R_5$ forment, conjointement avec l'atome d'azote auquel ils sont liés, un hétérocycle pipéridino ou morpholino, sous réserve que $R_4$ ou $R_6$ représente un atome d'hydrogène lorsque $R_7$ et $R_5$ ne représentent pas un atome d'hydrogène, ainsi que les sels de ces composés.

6. Procédé selon la revendication 4 ou 5, caractérisé par le fait que les composés de formule (II) sont choisis parmi la p-phénylènediamine, la 2-méthyl p-phénylènediamine, la méthoxyparaphénylènediamine, la chloroparaphénylènediamine, la 2,6-diméthylparaphénylènediamine, la 2,3-diméthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylpara phénylènediamine, la 3-méthyl 4-amino N,N-diéthyl aniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl] morpholine, la N-[(4'-amino)phényl]pipéridine, sous forme de base libre ou de sels.

7. Procédé selon la revendication 4 ou 5, caractérisé par le fait que les p-aminophénols sont choisis parmi le p-aminophénol, le 2-méthyl 4-amino phénol, le 3-méthyl 4-aminophénol, le 2-chloro 4-amino phénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 2,5-diméthyl 4-aminophénol, le 2-méthoxyméthyl 4-aminophénol.

8. Procédé selon l'une quelconque des revendications 1 à 3, caractérisé par le fait que les précurseurs de colorants d'oxydation sont des précurseurs de colorants d'oxydation de type ortho choisis parmi les ortho-aminophénols et les ortho-phénylènediamines.

9. Procédé selon l'une quelconque des revendications 1 à 8, caractérisé par le fait que l'agent oxydant est choisi parmi le peroxyde d'hydrogène, le peroxyde d'urée, les bromates de métaux alcalins, les persels.

10. Procédé selon l'une quelconque des revendications 1 à 9, caractérisé par le fait que le pH de la composition appliquée sur les fibres kératiniques est compris entre 3 et 6,9.

11. Procédé selon l'une quelconque des revendications 1 à 10, caractérisé par le fait que la composition utilisée pour la teinture des fibres kératiniques contient en plus des coupleurs hétérocycliques de la famille des 4-hydroxyindoles de formule (I), d'autres coupleurs choisis parmi les métadiphénols, les métaaminophénols, les métaphénylènediamines, les méta N-acylaminophénols, les métauréidophénols, les méta-carbalcoxyaminophénols, l'$\alpha$-naphtol, les coupleurs possédant un groupement méthylène actif choisis parmi les composés dicétoniques et les pyrazolones.

**12.** Procédé selon la revendication 11, caractérisé par le fait que les coupleurs sont choisis parmi le 2,4-dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-($\beta$-hydroxyéthyl)amino phénol, le 2-méthyl 5-N-($\beta$-mésylaminoéthyl)aminophénol, la 6-hydroxybenzomorpholine, le 2,4-diaminoanisole, le 2,4-diaminophénoxyéthanol, la 6-aminobenzomorpholine, le [2-N-($\beta$-hydroxyéthyl)amino 4-amino]-phénoxyéthanol, le 2-amino 4-N-($\beta$-hydroxyéthyl)aminoanisole, le (2,4-diamino)phényl-$\beta$, $\gamma$-dihydroxypropyléther, la 2,4-diaminophénoxyéthylamine, le 1,3-diméthoxy 2,4-diamino benzène, le 2-méthyl 5-aminophénol, le 2,6-diméthyl 3-aminophénol, le 3,4-méthylènedioxyphénol, la 3,4-méthylènedioxyaniline et leurs sels.

**13.** Procédé selon l'une quelconque des revendications 1 à 12, caractérisé par le fait que la composition contient des colorants directs et/ou des colorants d'oxydation rapides.

**14.** Procédé selon l'une quelconque des revendications 1 à 13, caractérisé par le fait que la composition contient des agents tensio-actifs anioniques, cationiques, non ioniques, amphotères ou leurs mélanges; des agents épaississants, des agents anti-oxydants et/ou tout autre adjuvant cosmétiquement acceptable.

**15.** Procédé selon l'une quelconque des revendications 1 à 14, caractérisé par le fait que le milieu approprié pour la teinture est constitué par de l'eau ou un mélange d'eau et d'un solvant choisi parmi les alcanols inférieurs en $C_2$-$C_4$, le glycérol, les glycols, les éthers de glycols, les alcools aromatiques ou leurs mélanges.

**16.** Agent de teinture des fibres kératiniques et en particulier des cheveux, caractérisé par le fait qu'il comporte au moins deux composants; un composant (A) constitué par une composition contenant dans un milieu approprié pour la teinture, un coupleur indolique répondant à la formule (I) tel que défini dans l'une quelconque des revendications 1 à 3 et un précurseur de colorant d'oxydation tel que défini dans l'une quelconque des revendications 1 et 4 à 8, un composant (B) constitué par une composition contenant dans un milieu approprié pour la teinture, un agent oxydant, le pH des composants (A) et (B) étant tel qu'après mélange dans des proportions de 90 à 10% pour le composant (A) et de 10 à 90% pour le composant (B), la composition résultante ait un pH inférieur à 7.

**17.** Agent selon la revendication 16, caractérisé par le fait que le composant (A) a un pH compris entre 3 et 10,5.

**18.** Agent selon la revendication 16 ou 17, caractérisé par le fait que le composant (A) contient les précurseurs de colorants par oxydation du type para et/ou ortho ainsi que les coupleurs, dans des proportions comprises entre 0,05 et 7% en poids par rapport au poids total du composant (A).

**19.** Agent selon l'une quelconque des revendications 16 à 18, caractérisé par le fait que la concentration en composés de formule (I) est comprise entre 0,01 et 4% en poids par rapport au poids total du composant A.

**20.** Agent selon l'une quelconque des revendications 16 à 19, caractérisé par le fait que le composant (A) contient des agents tensio-actifs dans des proportions de 0,1 à 55% en poids, des agents solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

**21.** Agent selon l'une quelconque des revendications 16 à 20, caractérisé par le fait que le composant (B) a un pH qui a une valeur minimum de 1 et inférieure à 7.

**22.** Procédé de teinture des fibres kératiniques et en particulier des cheveux, caractérisé par le fait qu'il comporte une première étage consistant à stocker un agent de teinture tel que défini dans l'une quelconque des revendications 16 à 21 et à procéder avant application au mélange des composants (A) et (B)

dans des proportions de 10 à 90% pour le composant (A) et de 90 à 10% pour le composant (B), de façon à obtenir une composition ayant un pH inférieur à 7 et d'appliquer ce mélange immédiatement après préparation sur les fibres kératiniques.

**23.** Dispositif à plusieurs compartiments ou kit de teinture, caractérisé par le fait qu'il comporte au moins deux compartiments dont un premier compartiment renferme le composant (A) tel que défini dans l'une quelconque des revendications 16 à 20, et le second compartiment renferme le composant (B) tel que défini dans les revendications 16 et 21.

**24.** Dispositif selon la revendication 23, caractérisé par le fait qu'il est équipé d'un moyen permettant de délivrer sur les cheveux le mélange souhaité des composants (A) et (B).

**25.** Procédé de teinture selon l'une quelconque des revendications 1 à 15, caractérisé par le fait que l'on applique sur les cheveux la composition et qu'on la laisse poser pendant 3 à 40 minutes, que l'on rince les cheveux et qu'on procède éventuellement à un shampooing avant un nouveau rinçage et séchage.

**26.** Utilisation comme coupleurs de dérivés du 4-hydroxyindole répondant à la formule (I) tels que définis dans l'une quelconque des revendications 1 à 3 pour la teinture en milieu acide des fibres kératiniques, en association avec des précurseurs de colorants d'oxydation.

**27.** Composition de teinture de fibres kératiniques, prête à l'emploi telle que mise en oeuvre dans le procédé selon l'une quelconque des revendications 1 à 15.

**28.** Composition selon la revendication 27, contenant le composé de formule I dans des proportions de 0,01 à 3,5% en poids par rapport au poids total de la composition.

## Claims

**1.** Method for dyeing keratinous fibres, in particular human keratinous fibres such as hair, characterised in that a composition containing, in a medium appropriate for dyeing, at least one coupler corresponding to the formula:

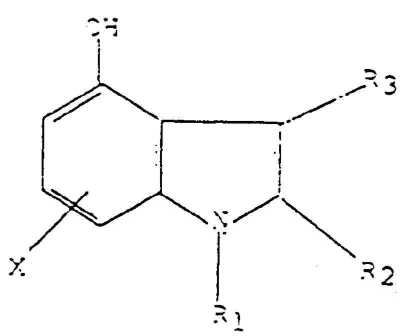

(I)

in which $R_1$ denotes a hydrogen atom or a $C_1$-$C_4$ alkyl radical; $R_2$ and $R_3$, which may be identical or different, denote a hydrogen atom, a $C_1$-$C_4$ alkyl radical, a carboxyl radical or an alkoxycarbonyl radical; and X denotes a hydrogen atom, a $C_1$-$C_4$ alkyl radical, a $C_1$-$C_{18}$ alkoxy radical, a halogen atom or an acetylamino group; at least one of the groups X, $R_1$, $R_2$ and $R_3$ being other than hydrogen;
as well as the salts of these compounds;

- at least one oxidation dye precursor; and
- at least one oxidising agent; is applied to said fibres, the pH of the composition applied to the fibres being less than 7.

**2.** Method according to Claim 1, characterised in that the compounds of formula (I) are chosen from the compounds in which the alkyl radical denotes methyl or ethyl and the alkoxycarbonyl radical denotes methoxycarbonyl or ethoxycarbonyl.

**3.** Method according to Claim 2, characterised in that the compounds of formula (I) are chosen from 4-hydroxy-5-methoxyindole, 4-hydroxy-2-ethoxycarbonyl-5-ethoxyindole, 4-hydroxy-2-methyl-5-ethoxyindole, 4-hydroxy-7-methoxy-2,3-dimethylindole, 4-hydroxy-5-methylindole, 4-hydroxy-5-ethoxyindole and 4-hydroxy-1-methyl-5-ethoxyindole.

**4.** Method according to any one of Claims 1 to 3, characterised in that the oxidation dye precursors are chosen from para-phenylenediamines, para-aminophenols and para heterocyclic precursors.

**5.** Method according to Claim 4, characterised in that the para-phenylenediamines are chosen from the compounds corresponding to the formula:

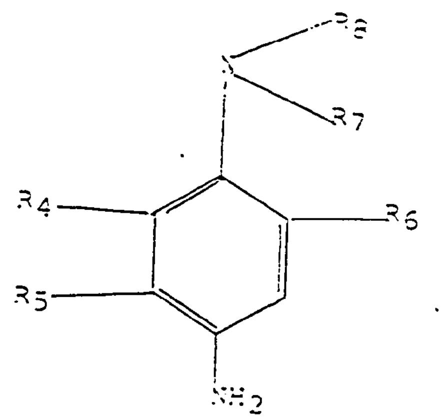

(II)

in which $R_4$, $R_5$ and $R_6$, which may be identical or different, represent a hydrogen or halogen atom, an alkyl radical having from 1 to 4 carbon atoms or an alkoxy radical having from 1 to 4 carbon atoms; and $R_7$ and $R_8$, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl, or morpholinoalkyl radical; these alkyl or alkoxy groups having from 1 to 4 carbon atoms, or $R_7$ and $R_8$ form, together with the nitrogen atom to which they are bonded, a piperidino or morpholino heterocycle, on condition that $R_4$ or $R_6$ represents a hydrogen atom when $R_7$ and $R_8$ do not represent a hydrogen atom, and the salts of these compounds.

**6.** Method according to Claim 4 or 5, characterised in that the compounds of formula (II) are chosen from p-phenylenediamine,2-methyl-p-phenylenediamine, methoxy-para-phenylenediamine, chloro-para-phenylenediamine, 2,6-dimethyl-para-phenylenediamine, 2,3-dimethyl-para-phenylenediamine, 2,5-dimethyl-para-phenylenediamine 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di-(β-hydroxyethyl)para-phenylenediamine, 3-methyl-4-amino-N,N-di-(β-hydroxyethyl)aniline, 3-chloro-4-amino-N,N-di-(β-hydroxyethyl)aniline, 4-amino-N,N-(ethyl, carbamylmethyl)aniline, 3-methyl-4-amino-N,N-(ethyl, carbamylmethyl)aniline, 4-amino-N,N-(ethyl,β-piperidinoethyl)aniline, 3-methyl-4-amino-N,N-(ethyl-β-piperidinoethyl)aniline, 4-amino-N,N-(ethyl,β-morpholinoethyl)aniline, 3-methyl-4-amino-N,N-(ethyl,β-morpholinoethyl)aniline, 4-amino-N,N-(ethyl,β-acetylaminoethyl)aniline, 4-amino-N-(β-methoxyethyl)aniline, 3-methyl-4-amino-N,N-(ethyl, β-acetylaminoethyl)aniline, 4-amino-N,N-(ethyl,β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine and N-[(4'-amino)phenyl]piperidine, in the form of the free base or of salts.

**7.** Method according to Claim 4 or 5, characterised in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol and 2-methoxymethyl-4-aminophenol.

**8.** Method according to any one of Claims 1 to 3, characterised in that the oxidation dye precursors are oxidation dye precursors of the ortho type chosen from ortho-aminophenols and ortho-phenylenediamines.

**9.** Method according to any one of Claims 1 to 8, characterised in that the oxidising agent is chosen from hydrogen peroxide, urea peroxide, alkali metal bromates and persalts.

10. Method according to any one of Claims 1 to 9, characterised in that the pH of the composition applied to the keratinous fibres is between 3 and 6.9.

11. Method according to any one of Claims 1 to 10, characterised in that the composition used for dyeing keratinous fibres contains, in addition to the heterocyclic couplers of the family of 4-hydroxyindoles of formula (I), other couplers chosen from meta-diphenols, meta-aminophenols, meta-phenylenediamines, meta-N-acylaminophenols, meta-ureidophenols, meta-carbalkoxyaminophenols, α-naphthol and couplers containing an active methylene group chosen from diketone compounds and pyrazolones.

12. Method according to Claim 11, characterised in that the couplers are chosen from 2,4-dihydroxyphenoxyethanol, 2,4-dihydroxyanisole, meta-aminophenol, resorcinol, resorcinol monomethyl ether, 2-methylresorcinol, pyrocatechol, 2-methyl-5-N-(β-hydroxyethyl)aminophenol, 2-methyl-5-N-(β-mesylaminoethyl)-aminophenol, 6-hydroxybenzomorpholine, 2,4-diaminoanisole, 2,4-diaminophenoxyethanol, 6-aminobenzomorpholine, [2-N-(β-hydroxyethyl)amino-4-amino]-phenoxyethanol, 2-amino-4-N-(β-hydroxyethyl)-aminoanisole, (2,4-diamino)phenyl-β,γ-dihydroxypropyl ether, 2,4-diaminophenoxyethylamine, 1,3-dimethoxy-2,4-diaminobenzene, 2-methyl-5-aminophenol, 2,6-dimethyl-3-aminophenol, 3,4-methylenedioxyphenol and 3,4-methylenedioxyaniline and their salts.

13. Method according to any one of Claims 1 to 12, characterised in that the composition contains direct dyes and/or rapid oxidation dyes.

14. Method according to any one of Claims 1 to 13, characterised in that the composition contains anionic, cationic, nonionic or amphoteric surface-active agents or their mixtures; thickeners, antioxidants and/or any other cosmetically acceptable adjuvant.

15. Method according to any one of Claims 1 to 14, characterised in that the medium appropriate for dyeing consists of water or a mixture of water and a solvent chosen from $C_2$-$C_4$ lower alkanols, glycerol, glycols, glycol ethers and aromatic alcohols or their mixtures.

16. Agent for dyeing keratinous fibres and in particular hair, characterised in that it comprises at least two components: a component (A) consisting of a composition containing, in a medium appropriate for dyeing, an indole coupler corresponding to the formula (I) as defined in any one of Claims 1 to 3 and an oxidation dye precursor as defined in any one of Claims 1 and 4 to 8, and a component (B) consisting of a composition containing an oxidising agent in a medium appropriate for dyeing, the pH of the components (A) and (B) being such that, after mixing in proportions of 90 to 10% in respect of component (A) and of 10 to 90% in respect of component (B), the resulting composition has a pH of less than 7.

17. Agent according to Claim 16, characterised in that the component (A) has a pH of between 3 and 10.5.

18. Agent according to Claim 16 or 17, characterised in that the component (A) contains the oxidation dye precursors of the para and/or ortho type and also the couplers, in proportions of between 0.5 and 7% by weight relative to the total weight of the component (A).

19. Agent according to any one of Claims 16 to 18, characterised in that the concentration of compounds of formula (I) is between 0.01 and 4% by weight relative to the total weight of the component (A).

20. Agent according to any one of Claims 16 to 19, characterised in that the component (A) contains surface-active agents in proportions of 0.1 to 55% by weight, solvents in addition to water in proportions of between 0.5 and 40% by weight, thickeners in proportions of between 0.1 and 5% by weight, antioxidants in proportions of between 0.02 and 1.5% by weight and/or any other cosmetically acceptable adjuvant.

21. Agent according to any one of Claims 16 to 20,. characterised in that the component (B) has a pH which has a minimum value of 1 and less than 7.

22. Method for dyeing keratinous fibres and in particular hair, characterised in that it comprises a first step consisting in storing a dyeing agent as defined in any one of Claims 16 to 21 and, before application, in mixing the components (A) and (B) in proportions of 10 to 90% in respect of component (A) and of 90 to 10% in respect of component (B), so as to obtain a composition having a pH of less than 7, and applying this mixture immediately after preparation to the keratinous fibres.

23. Multi-compartment device or dyeing kit, characterised in that it comprises at least two compartments, a first compartment of which contains the component (A) as defined in any one of Claims 16 to 20 and the second compartment contains the component (B) as defined in Claims 16 and 21.

24. Device according to Claim 23, characterised in that it is fitted with means permitting the desired mixture of components (A) and (B) to be delivered onto the hair.

25. Dyeing method according to any one of Claims 1 to 15, characterised in that the composition is applied to the hair and in that it is left on the hair for 3 to 40 minutes, in that the hair is rinsed and in that shampooing is optionally carried out before rinsing again and drying.

26. Use of 4-hydroxyindole derivatives corresponding to the formula (I) as defined in any one of Claims 1 to 3 as couplers for dyeing keratinous fibres in an acid medium, in combination with oxidation dye precursors.

27. Ready-to-use composition for dyeing keratinous fibres, as used in the method according to any one of Claims 1 to 15.

28. Composition according to Claim 27, containing the compound of formula (I) in proportions of from 0.01 to 3.5% by weight relative to the total weight of the composition.

## Patentansprüche

1. Färbeverfahren für Keratinfasern, insbesondere menschliche Keratinfasern, wie Haare, dadurch gekennzeichnet, daß man auf die Fasern eine Zusammensetzung appliziert, die in einem zur Färbung geeigneten Milieu mindestens einen Kuppler der Formel:

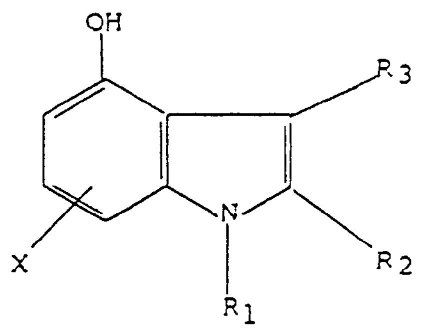

(I)

worin $R_1$ ein Wasserstoffatom oder ein $C_1$-$C_4$-Alkylradikal bedeutet; $R_2$ und $R_3$, die identisch oder verschieden sein können, ein Wasserstoffatom, ein $C_1$-$C_4$-Alkylradikal, ein Carboxylradikal oder ein Alkoxycarbonylradikal bedeuten; X ein Wasserstoffatom, ein $C_1$-$C_4$-Alkylradikal, ein $C_1$-$C_{18}$-Alkoxyradikal, ein Halogenatom oder eine Acetylaminogruppe bedeutet; wobei mindestens eine der Gruppen X, $R_1$, $R_2$, $R_3$ von Wasserstoff verschieden ist; sowie die Salze dieser Verbindungen;
- mindestens einen Oxidationsfarbstoffvorläufer;
- mindestens ein Oxidationsmittel enthält,

und der pH-Wert der auf den Fasern aplizierten Zusammensetzung unterhalb von 7 liegt.

2. Verfahren nach Anspruch 1, dadurch gekennzeichnet, daß die Verbindungen der Formel (I) ausgewählt sind unter den Verbindungen, in denen das Alkylradikal Methyl oder Ethyl bedeutet; und das Alkoxycarbonylradikal Methoxy- oder Ethoxycarbonyl bedeutet.

3. Verfahren nach Anspruch 2, dadurch gekennzeichnet, daß die Verbindungen der Formel (I) ausgewählt sind unter 4-Hydroxy-5-methoxyindol, 4-Hydroxy-2-ethoxycarbonyl-5-ethoxyindol, 4-Hydroxy-2-methyl-5-ethoxyindol, 4-Hydroxy-7-methoxy-2,3-dimethylindol, 4-Hydroxy-5-methylindol, 4-Hydroxy-5-ethoxyindol, 4-Hydroxy-1-methyl-5-ethoxyindol.

4. Verfahren nach einem der Ansprüche 1 bis 3, dadurch gekennzeichnet, daß die Oxidationsfarbstoffvorläufer ausgewählt sind unter den Paraphenylendiaminen, para-Aminophenolen, den para-heterocyklischen Vorläufern.

5. Verfahren nach Anspruch 4, dadurch gekennzeichnet, daß die Paraphenylendiamine ausgewählt sind unter den Verbindungen der Formel:

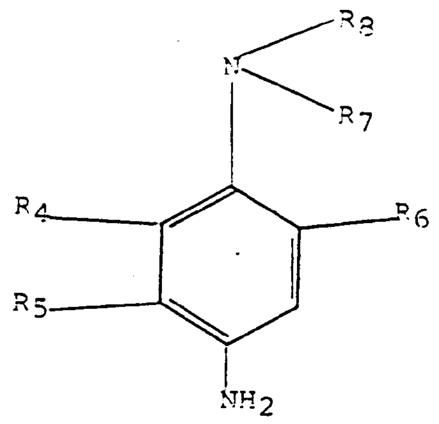

(II)

worin $R_4$, $R_5$ und $R_6$, die identisch oder verschieden sein können, ein Wasserstoffatom oder Halogenatom, ein Alkylradikal mit 1 bis 4 Kohlenstoffatomen oder ein Alkoxyradikal mit 1 bis 4 Kohlenstoffatomen bedeuten; $R_7$ und $R_5$, die identisch oder verschieden sein können, ein Wasserstoffatom, ein Alkylradikal, Hydroxyalkyl, Alkoxyalkyl, Carbamyolalkyl, Mesylaminoalkyl, Acetylaminoalkyl, Ureidoalkyl, Carbalkoxyaminoalkyl, Piperidinoalkyl oder Morpholinoalkyl bedeuten, wobei die Alkyl- oder Alkoxygruppen 1 bis 4 Kohlenstoffatome besitzen, oder $R_7$ und $R_5$ zusammen mit dem Stickstoffatom, an das sie gebunden sind, auch einen Piperidin- oder Morpholin-Heterocyklus bilden, mit der Maßgabe, daß $R_4$ oder $R_5$ ein Wasserstoffatom bedeuten, wenn $R_7$ und $R_5$ kein Wasserstoffatom bedeuten, sowie die Salze dieser Verbindungen.

6. Verfahren nach Anspruch 4 oder 5, dadurch gekennzeichnet, daß die Verbindungen der Formel (II) ausgewählt sind unter p-Phenylendiamin, 2-Methyl-p-Phenylendiamin, Methoxyparaphenylendiamin, Chlorparaphenylendiamin, 2,6-Dimethylparaphenylendiamin, 2,3-Dimethylparaphenylendiamin, 2,5-Dimethylparaphenylendiamin, 2-Methyl-5-methoxyparaphenylendiamin, 2,6-Dimethyl-5-methoxyparaphenylendiamin, N,N-Dimethyl-paraphenylendiamin, 3-Methyl-4-amino-N,N-diethylanilin, N,N-Di-(β-hydroxyethyl)-paraphenylendiamin, 3-Methyl-4-amino-N,N-di-(β-hydroxyethyl)-anilin, 3-Chlor-4-amino-N,N-di-(β-hydroxyethyl)-anilin, 4-Amino-N,N-(ethyl,carbamoylmethyl)-anilin, 3-Methyl-4-amino-N,N-(ethyl,carbamoylmethyl)-anilin, 4-Amino-N,N-(ethyl,β-piperidinoethyl)-anilin, 3-Methyl-4-amino-N,N-(ethyl,β-piperidinoethyl)-anilin, 4-Amino-N,N-(ethyl,β-morpholinoethyl)-anilin, 3-Methyl-4-amino-N,N-(ethyl,β-morpholinoethyl)-anilin, 4-Amino-N,N-(ethyl,β-acetylaminoethyl)-anilin, 4-Amino-N-(β-methoxyethyl)-anilin, 3-Methyl-4-amino-N,N-(ethyl,β-acetylaminoethyl)-anilin, 4-Amino-N,N-(ethyl,β-mesylaminoethyl)-anilin, 3-Methyl-4-amino-N,N-(ethyl,β-mesylaminoethyl)-anilin, 4-Amino-N,N-(ethyl,β-sulfoethyl)-anilin, 3-Methyl-4-amino-N,N-(ethyl,β-sulfoethyl)-anilin, N-(4-aminophenyl)-morpholin, N-(4-Amino-phenyl)-piperidin, in Form der freien Base oder der Salze.

7. Verfahren nach Anspruch 4 oder 5, dadurch gekennzeichnet, daß die p-Aminophenole ausgewählt sind unter p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol.

8. Verfahren nach einem der Ansprüche 1 bis 3, dadurch gekennzeichnet, daß die Oxidationsfarbstoffvorläufern vom ortho-Typ sind, ausgewählt unter den ortho-aminophenolen und den ortho-Phenylendiaminen.

9. Verfahren nach einem der Ansprüche 1 bis 8, dadurch gekennzeichnet, daß das Oxidationsmittel ausgewählt ist unter Wasserstoffperoxid, Harnstoffperoxid, den Alkalimetallbromaten, den Persalzen.

10. Verfahren nach einem der Ansprüche 1 bis 9, dadurch gekennzeichnet, daß der pH-Wert der auf den Keratinfasern applizierten Zusammensetzung zwischen 3 und 6,9 liegt.

**11.** Verfahren nach einem der Ansprüche 1 bis 10, dadurch gekennzeichnet, daß die zur Färbung der Keratinfasern verwendete Zusammensetzung zusätzlich zu den heterocyklischen Kupplern der Familie der 4-Hydroxyindole der Formel (I) andere Kuppler enthält, ausgewählt unter den Metadiphenolen, den Metaaminophenolen, den Metaphenylendiaminen, den meta-N-Acylaminophenolen, den meta-Ureidophenolen, den meta-Carbalkoxyaminophenolen, α-Naphtol, den eine aktive Methylengruppe aufweisenden Kupplern, ausgewählt unter den Diketonverbindungen und den Pyrazolonen.

**12.** Verfahren nach Anspruch 11, dadurch gekennzeichnet, daß die Kuppler ausgewählt sind unter 2,4-Dihydroxyphenoxyethanol, 2,4-Dihydroxyanisol, Metaaminophenol, Resorcin, Resorcinmonomethylether, 2-Methylresorcin, Brenzkatechin, 2-Methyl-5-N-(β-hydroxyethyl)-aminophenol, 2-Methyl-5-N-(β-mesylaminoethyl)-aminophenol, 6-Hydroxybenzomorpholin, 2,4-Diaminoanisol, 2,4-Diaminophenoxyethanol, 6-Aminobenzomorpholin, 2-(N-β-hydroxyethylamino)-4-amino-phenoxyethanol, 2-Amino-4-(N-β-hydroxyethylamino)-anisol, (2,4-Diaminophenyl)-β, γ-dihydroxypropylether, 2,4-Diaminophenoxyethylamin, 1,3-Dimethoxy-2,4-diaminobenzol, 2-Methyl-5-aminophenol, 2,6-Dimethyl-3-aminophenol, 3,4-Methylendioxyphenol, 3,4-Methylendioxyanilin und ihren Salzen.

**13.** Verfahren nach einem der Ansprüche 1 bis 12, dadurch gekennzeichnet, daß die Zusammensetzung Direktfarbstoffe und/oder schnelle Oxidationsfarbstoffe enthält.

**14.** Verfahren nach einem der Ansprüche 1 bis 13, dadurch gekennzeichnet, daß die Zusammensetzung anionische, kationische, nicht-ionische, amphotere oberflächenaktive Mittel oder ihre Mischungen, Verdickungsmittel, Antioxidationsmittel und/oder irgendeinen anderen kosmetisch annehmbaren Zusatzstoff enthält.

**15.** Verfahren nach einem der Ansprüche 1 bis 14, dadurch gekennzeichnet, daß das zur Färbung geeignete Milieu aus Wasser oder einer Mischung aus Wasser und einem Losungsmittel ausgewählt unter den $C_2$-$C_4$-Niederalcanolen, Glycerin, Glykolen, Glykolethern, aromatischen Alkoholen oder ihren Mischungen besteht.

**16.** Färbemittel für Keratinfasern und insbesondere für Haare, dadurch gekennzeichnet, daß es mindestens zwei Komponenten enthält, wobei eine Komponente (A) aus einer Zusammensetzung besteht, die in einem zur Färbung geeigneten Milieu einen Indolkuppler der Formel (I), wie er in einem der Ansprüche 1 bis 3 definiert ist, und einen Oxidationsfarbstoffvorläufer, wie er in einem der Ansprüche 1 und 4 bis 8 definiert ist, besteht, und die Komponente (B) aus einer Zusammensetzung enthält, die in einem zur Färbung geeigneten Milieu ein Oxidationsmittel enthält, wobei der pH-Wert der Komponenten (A) und (B) ein solcher ist, daß nach dem Mischen in Anteilen von 90 bis 10 % für Komponente (A) und von 10 bis 90 % für Komponente (B) die resultierende Mischung einen pH-Wert von unterhalb 7 besitzt.

**17.** Mittel nach Anspruch 16, dadurch gekennzeichnet, daß die Komponente (A) einen pH-Wert zwischen 3 und 10,5 aufweist.

**18.** Mittel nach Anspruch 16 oder 17, dadurch gekennzeichnet, daß die Komponente (A) Oxidationsfarbstoffvorläufer vom para- und/oder ortho-Typ sowie die Kuppler in Anteilen zwischen 0,05 und 7 Gew.-%. bezogen auf das Gesamtgewicht der Komponente (A), enthält.

**19.** Mittel nach einem der Ansprüche 16 bis 18, dadurch gekennzeichnet, daß die Konzentration der Verbindungen der Formel (I) zwischen 0,01 und 4 Gew.-%, bezogen auf das Gesamtgewicht der Komponente (A) liegt.

**20.** Mittel nach einem der Ansprüche 16 bis 19, dadurch gekennzeichnet, daß die Komponente (A) oberflächenaktive Mittel in Anteilen von 0,1 bis 55 Gew.-%, zusätzlich zum Wasser Lösungsmittel in Anteilen zwischen 0,5 und 40 Gew.-%, Verdickungsmittel in Anteilen zwischen 0,1 und 5 Gew.-%, Antioxidationsmittel in Anteilen zwischen 0,02 und 1,5 Gew.-%, und/oder irgendeinen anderen kosmetisch annehmbaren Zusatzstoff enthält.

**21.** Mittel nach einem der Ansprüche 16 bis 20, dadurch gekennzeichnet, daß die Komponente (B) einen pH-Wert aufweist, der einen Minimalwert von 1 besitzt und unterhalb von 7 liegt.

**22.** Färbeverfahren für Keratinfasern, insbesondere Haare, dadurch gekennzeichnet, daß es eine erste Stufe

umfaßt, in der man ein nach einem der Ansprüche 16 bis 21 definiertes Färbemittel lagert und vor der Applikation eine Mischung der Komponenten (A) und (B) in Anteilen von 10 bis 90 % für die Komponente (A) und von 90 bis 10 % für die Komponente (B) so herstellt, daß man eine Zusammensetzung mit einem pH-Wert von unterhalb 7 erhält, und die Mischung sofort nach der Herstellung auf die Keratinfasern appliziert.

**23.** Vorrichtung aus mehreren Kompartimenten oder Färbekit, dadurch gekennzeichnet, daß sie mindestens zwei Kompartimente umfaßt, deren erstes Kompartiment die Komponente (A) nach einem der Ansprüche 16 bis 20 enthält, und das zweite Kompartiment die Komponente (B) nach den Ansprüchen 16 und 21 enthält.

**24.** Vorrichtung nach Anspruch 23, dadurch gekennzeichnet, daß sie mit einer Einrichtung ausgestattet ist, die es erlaubt, die gewünschte Mischung der Komponenten (A) und (B) auf die Haare aufzubringen.

**25.** Färbeverfahren nach einem der Ansprüche 1 bis 15, dadurch gekennzeichnet, daß man die Zusammensetzung auf die Haare appliziert, während 3 bis 40 Minuten einwirken läßt, die Haare spült und gegebenenfalls vor einer erneuten Spülung shampooniert, und trocknet.

**26.** Verwendung der 4-Hydroxyindolderivate der Formel (I) nach einem der Ansprüche 1 bis 3 als Kuppler zur Färbung von Keratinfasern in einem sauren Milieu zusammen mit Oxidationsfarbstoffvorläufern.

**27.** Färbezusammensetzung für Keratinfasern zur Verwendung in einem Verfahren gemäß einem der Ansprüche 1 bis 15.

**28.** Zusammensetzung nach Anspruch 27, dadurch gekennzeichnet, daß sie die Verbindung der Formel (I) in Anteilen von 0,01 bis 3,5 Gew.-%, bezogen auf das Gesamtgewicht der Zusammensetzung, enthält.